(19) Europäisches Patentamt
European Patent Office
Office européen des brevets

(11) **EP 2 457 598 B1**

(12) **EUROPEAN PATENT SPECIFICATION**

(45) Date of publication and mention
of the grant of the patent:
**08.10.2014 Bulletin 2014/41**

(51) Int Cl.:
**A61L 15/26** *(2006.01)*          **A61L 15/64** *(2006.01)*
**A61L 31/06** *(2006.01)*          **A61L 31/14** *(2006.01)*

(21) Application number: **10191049.5**

(22) Date of filing: **12.11.2010**

(54) **Resorbable membrane**

Resorbierbare Membran

Membrane résorbable

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**30.05.2012 Bulletin 2012/22**

(73) Proprietor: **nolax AG
6203 Sempach Station (CH)**

(72) Inventors:
 • **Blume, Jessica
  8050 Zürich (CH)**
 • **Buser, Stephan
  6210 Sursee (CH)**
 • **Dobmann, Andreas
  6208 Oberkirch (CH)**
 • **Zimmermann, Erika
  6374 Buochs (CH)**

(74) Representative: **Hepp Wenger Ryffel AG
Friedtalweg 5
9500 Wil (CH)**

(56) References cited:
 **EP-A1- 2 179 749     WO-A1-99/47072
 US-A- 5 135 964**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to resorbable membranes for medical use according to claim 1 as well as to a kit comprising resorbable membranes according to claim 8.

Membranes for medical use are used to separate internal body tissues or organs, to protect the body against external influences or to guide tissue regeneration.

A separation of tissues or organs may become important after surgery. As an example, a membrane is placed in the cavity after tooth extraction in order to avoid ingrowths of fibroblasts and cells of the gingiva into the jawbone.

Examples for uses as protection from external influences are topical applications, i.e. on the surface of the skin, especially for the treatment of burns and skin transplants. In both cases the main function of the membrane is to protect the wound from invading microorganisms. Thereby the permeability of the membrane for oxygen and humidity must be guaranteed.

Guided tissue regeneration is another area of application for membranes, for example in the treatment of periodontal diseases, the regeneration of gum tissue or for bone and nerve regeneration.

For the above uses non-resorbable membranes based on biocompatible polymers were initially commercialised (Acikel C., Eren F., Ergun O., Celikoz B., Topical Treatment of Toxic Epidermal Necrolysis using Omiderm® and Glycerol-Preserved Human Cadaver Skin, Annals of Burns and Fire Disasters, Vol XV, June 2002). In particular, PTFE membranes were considered as the gold standard for a long time as they have a good biocompatibility and exhibit excellent cell adhesion properties.

The big disadvantage of such non-resorbable membranes is the need to remove them. In particular for internal use this entails a second surgical intervention which may slow down the healing process, increases the treatment costs and presents a risk to the patient. Therefore, the use of resorbable membranes is advantageous. This is also the case for topical applications such as for the treatment of burns, since it spares the patient from painful replacements of the membrane.

Resorbable membranes may be either from a synthetic or a natural origin. Natural absorbable membranes, most of which consist of proteins (collagen) and polysaccharides have the big disadvantage of high production costs (Uhlig C., Rapp M., Hartmann B., Hierlemann H., Planck H., Dittel K.-K., Suprathel® - An innovative, resorbable skin substitue for treatment of burn victims, Burns 33 (2007), 221-229). Moreover, the use of resorbable membranes from natural sources carries the risk of transmitting pathogens to the patient.

Current synthetic absorbable membranes are mostly based on biodegradable polymers such as poly-lactic acid (PLA). For example, DE 19940977 A1 describes a film made of an absorbable polymer composition comprising a poly-(L-lactide)-derivative supplemented with osteoconductive materials such as tricalcium phosphate for lining a bone cavity.

US 6,706,058 describes a resorbable membrane on the basis of a terpolymer from lactide, trimethylene carbonate and $\varepsilon$-caprolactone, in which lactide is the main component. This membrane is reported to show optimal properties for the treatment of burns. In addition, the membrane is transparent, which allows an evaluation of the underlying tissue, without the need to remove the membrane.

WO 01/67987 teaches the use of a micro-membrane comprising a co-polymer, preferably L-lactide-co-D,L-lactide, for the prophylactic separation of internal organs or tissues. The use of such a membrane reduces the formation of post-surgical adhesions between a post surgical site and adjacent surrounding tissue.

The disadvantage common to these membranes is the use of lactic acid-based polymers. Degradation of such polymers leads to the formation of acids, thus leading to a local acidification at the application site (Kroeze R.J., Helder M.N., Govaert L.E., Smit T.H., Biodegradable Polymers in Bone Tissue Engineering, Materials 2009, 2, 833-856). This leads to an increased level of cell toxicity in the adjacent tissue which may slow down or even stop the healing process. This effect is enhanced in cases where the membrane shows a rapid absorption rate.

WO 99/47072 A1 discloses implants with a coating and breathable membranes comprising a hydrophilic polyether polyurethane produced using a diol and a diisocyanate.

One object of the present invention is therefore to avoid the disadvantages of the membranes known in the state of the art and especially to provide a membrane which is biocompatible and resorbable and which does not lead to degradation products which negatively influence the healing process. This objective is achieved with a membrane according to claim 1.

A resorbable membrane according to the present invention is produced or is producible from a composition comprising:

a) at least one polyol component comprising at least one compound with at least 2, but preferable 2, 3 or 4 hydroxyl groups;

b) at least one polyisocyanate component or at least one polyurethane pre-polymer comprising at least one compound with at least 2, but preferably 2, 3 or 4 isocyanate groups;

c) an additive comprising at least one of the following compounds: polyvinylpyrrolidone, polyvinylpolypyrrolidone, a copolymer of polyvinylpyrrolidone with vinylacetate, vinylimidazole or vinylcarpolactame or mixtures thereof.

A membrane as understood in the present application is a plane, uninterrupted three-dimensional structure having a maximal thickness of 1000 $\mu$m. Further, the membrane has to be permeable to gases, especially vapour, but impermeable to other substances, especially to micro-organisms. For this, the membrane preferably does not have pores which allow the passage of bacteria with a diametric size between 10 and 0.5 micrometers. Ideally, the passage of viruses with an average diametric size between 0.1 and 0.01 micrometers is also inhibited.

The composition is degradable by the body. A polyurethane membrane is resorbable by the body if the membrane can be disintegrated trough hydrolysis, oxidation or enzymatic cleavage. Hydrolysis occurs when the membrane is exposed to a water containing media, especially wound exudate. Oxidation reactions occur through the action of free oxygen radicals which may be released by macrophages during an inflammation reaction. Macrophages also secrete the enzyme cholesterol esterase which cleaves the ester bonds of the resorbable membrane. However the main resorption mechanism of resorbable biopolymers is believed to be hydrolysis (S.A. Guelcher, Biodegradable Polyurethanes: Synthesis and Applications in Regenerative Medicine, Tissue engineering: Part B, 2008, volume 14, no. 1; E.M. Christenson, S. Patel, J.M. Anderson, A. Hiltner, Enzymatic Degradation of Poly(ether urethane) and poly (carbonate urethane) by cholesterol esterase, Biomaterials, 2006, vol. 23, 3920 - 3926).

By using the composition according to the present invention, a membrane may be produced which does not lead to decomposition products which will alter the chemical properties of the surrounding tissue, especially in terms of pH. Further, the degradation products do not exhibit any toxicity.

Further, a membrane according to the present invention is transparent. This allows monitoring the healing process without the need to remove the membrane.

By addition of additive c. and by appropriate choice of the polyol component, the membrane will gel or expand upon contact with wound exudate. This gelling and expansion capacity enhances the adhesion of the membrane to wounds or organs. Further it increases the shock absorbance capacity of the membrane.

A particular advantage of the use of polyvinylpyrrolidone as additive c. is that it can bind proteins. By this binding the diffusion of growth factors will be hindered and free matrix metalloproteases (MMPs) will be bound, so that the concentration of these molecules will be lowered in the area of the growing cells. This leads to a positive influence on wound healing.

Surprisingly, it has been found that by using polyvinylpyrrolidone in the resorbable membrane, the adhesion of bacteria and biofilm formation can be reduced while adhesion of eukaryotic cells is not hindered. Without restricting the invention on any theory, it is believed that for a successful wound healing an intermediate extra cellular matrix of proteins has to be formed first which serves as an adhesion matrix for the cells. Formation of this intermediate extra cellular matrix is probably positively influenced by the polyvinylpyrrolidone.

Further advantages associated to the use of polyvinylpyrrolidone in resorbable membranes are its good biological compatibility as well as the lack of toxicity.

Additive c. alternatively may also comprise co-polymers of vinylpyrrolidone with vinylacetate, vinylimidazole or vinylcaprolactam (e.g. available under the trade name Luvitec VA64W, VA64, VPI55K72W and VPC55K65W from BASF). Most preferably a co-polymer of vinylpyrrolidone and vinylimidazol is used, since the imidazol structure enhances the adhesion to proteins.

The polyol component comprises at least one compound with at least two or more hydroxyl groups. Preferably, the polyol component comprises a mixture of two or more compounds with at least two or more hydroxyl groups. Preferred compounds are hydroxyl terminated poly ethers like $\alpha,\omega$-dihydroxy poly(oxyethylene), $\alpha,\omega$-dihydroxy poly(1,2-ethyleneoxide), $\alpha,\omega$-dihydroxy poly(1,2-propyleneoxide), $\alpha,\omega$-dihydroxy poly(1,3-trimethyleneoxide), $\alpha,\omega$-dihydroxy poly(1,4-tetramethyleneoxide), $\alpha,\omega$-dihydroxy poly(methyleneoxy-1,2- ethyleneoxide) and the like as well as copolymers thereof, preferably having molar masses of up to 15'000 g/mol; hydroxy terminated aliphatic polycarbonates like $\alpha,\omega$- dihydroxy poly(ethylenecarbonate), $\alpha,\omega$-dihydroxy poly(1,2-propylenecarbonate), $\alpha,\omega$- dihydroxy poly(1,3-propylenecarbonate), $\alpha,\omega$-dihydroxy poly(tetramethylenecarbonate), $\alpha,\omega$-dihydroxy poly(hexamethylenecarbonate) and the like as well as co-polymers thereof, preferably having molar masses of up to 15'000 g/mol; poly anhydrides from dicarbonic acids, like malonic acid, succinic acid, glutaric acid and the like as well as co-polymer thereof, preferably with molar masses up to 15000 g/mol; low molecular two- or polyvalent alcohols like glycol, polyethylene glycol, 1,2- propylene glycol, 1,3- propylene glycol, butane diol, pentane diol, hexane diol and long-chain linear or branched aliphatic diols, glycerol, triethanol amine, pentaerythritol, 2,2-bis(hydroxymethyl)propanol and the like; amino acid dimers, trimers and oligomers containing hydroxyl groups, e.g. from tyrosine and/or serine; as well as sugar alcohols like sorbitol and other natural compounds or derivatives of natural compounds having at least two hydroxyl groups. Further polyestertriols like castor oil and sulphonated castor oil may be used.

Preferably polyestertriols are added to the polyol component. This will lead to a higher polymerisation density within the polyurethane. Most preferably sulphonated castor oil is added.

Preferably, polyesters with hydroxyl groups are comprised in the polyol component. Examples of such compounds are poly caprolactonediol and poly caprolactonetriol (e.g. available under the trade name Capa from Solvay). Further examples are $\alpha,\omega$-dihydroxy poly(D,L-lactide), $\alpha,\omega$-dihydroxy poly(D-lactide), $\alpha,\omega$-dihydroxy poly(L-lactide), $\alpha,\omega$-dihydroxy poly(glycolide), $\alpha,\omega$-dihydroxy poly (hydroxybutyrate) and other aliphatic polyester and their co-polymers including segmented block co-polymers of polyether and polyester segments, like they are obtainable from reacting high molecular polyesters with hydroxyl terminated poly(alkyleneglycols), as well as mixtures of such polyols.

Most preferably, compounds are used which are biocompatible and which may be resorbed by the body. Examples of such compounds are poly($\varepsilon$-caprolactone) (PCL), poly($\varepsilon$-caprolactone-coglycolide-co-DL-lactide), branched and unbranched poly ethylene glycole (PEG), PCL-b-PEG-b-PCL, ($\alpha,\omega$-dihydroxy-oligo(((R)-3-hydroxybutyrate-Co-(R)-3-hydroxyvalerate)-block-ethylene glycol).

For the production of the resorbable membrane polyurethane pre-polymers may be used. In general the pre-polymers used for the production of a resorbable membrane according to the present invention preferably have a molar mass of between 400 and 15'000, more preferably of between 400 and 10'000, and most preferably between 400 and 1'000.

As an alternative to the polyurethane pre-polymers a polyisocyanate component with at least one compound with at least two isocyanate groups may be used in the composition. The polyisocyanate component preferably comprises at least one biocompatible aliphatic polyisocyanate or at least one compound derived from a biocompatible polyamine. Preferred compounds are: a substituted or unsubstituted alkylenediisocyanate with 3 to 12 carbon atoms like hexamethylenediisocyanate or lysinediisocyanate; substituted or unsubstituted cycloalkylenediisocyanates with 5 to 15 carbon atoms like cyclohexylenediisocyanate; substituted or unsubstituted alkylcycloalkylenediisocyanate with 6 to 18 carbon atoms like isophoronediisocyanate; substituted or unsubstituted aromatic diisocyanates like p-phenylenediisocyanante, toluyldiisocyanate (all isomers and mixtures thereof), 4,4'-diphenylmethanediisocyanate; as well as isomers, trimers, higher oligomers, uretdions, cyanurates and isocyanurates of these diisocyanates and the like.

Most preferably hexamethylenediisocyanat, 1,6-diisocyanatohexane (HDI), 1,4-diisocyanatobutane (BDI), isophoronediisocyanate (IPDI), dicyclohexylmethanediisocyanate (H12MDI), lysinmethylesterdiisocyanate (LDI) or 4.4'-diphenylmethane-diisocyanate (MDI) is used.

Moreover, the composition may also comprise catalysts which are non-toxic and which show no increased toxicity when resorbed by the body. The catalysts should be able to catalyze the reaction between hydroxyl and isocyanate groups. In a composition of the present invention an organometallic catalyst and/or a tertiary amine catalyst is preferably used. Most preferably, a bismuth catalyst and/or 2'2'-dimorpholinyldiethylether is used. The concentration of the catalysts preferably is in the range of 0.01 - 1.00 weight percent.

Further components like emulsifiers and the like may also be comprised in the composition.

The membrane preferably has a thickness of between 10 $\mu$m and 1000 $\mu$m, more preferably between 20 $\mu$m and 500 $\mu$m, most preferably between 50 $\mu$m and 300 $\mu$m. Thinner membranes have a higher moisture vapour transmission rate than thicker membranes. Thus, the thickness of the membrane has to be chosen according to the intended application.

The at least one polyol component is preferably present in an amount between 1 and 96% by weight, more preferably between 30 and 60% by weight, most preferably between 35 and 50% by weight.

Preferably the composition further comprises between 4 and 60 weight percent, more preferably between 40 and 60 weight percent, and most preferably between 45 and 55 weight percent of the at least one isocyanate compound or the polyurethane prepolymer.

The additive c. is preferably present in an amount of between 0.001 and 10% by weight, preferably between 1 and 4% by weight.

Additive c., especially polyvinylpyrrolidone, is added in a relatively small concentration to the composition wherein, after polymerisation of the membrane, it will be mostly distributed within and on the surface of the membrane in small "islands". This leads to areas where the concentration of additive c., especially polyvinylpyrrolidone, is partially increased. Thus, even if only small concentrations of additive c., especially polyvinylpyrrolidone, are used in the composition, areas with a high enough concentration to lead to a biological activity will be present.

The polyvinylpyrrolidone used as component c. may comprise polyvinylpyrrolidone with a defined average molar mass or alternatively may comprise a mixture of polyvinylpyrrolidones with different average molar masses. Preferably polyvinylpyrrolidone with an average molar mass of between 7'000 to 1'000'000 or a mixture thereof is used as dispersion. Most preferably, polyvinylpyrrolidone with an average molar mass of between 40'000 and 60'000 is used (e.g. available under the trade name Luvitec K30 from BASF).

The at least one polyol component preferably comprises at least one polyethyleneglycol. Polyethyleneglycol has the advantage that it is not toxic and that its decomposition products do not alter the pH value or the ionic strength of the surrounding tissue.

In an especially preferred embodiment of the present invention the composition additionally comprises at least one polycaprolactone, most preferably at least one poly (ε-caprolactone). The at least one polycaprolactone may be branched or unbranched and preferably has an average molar mass of between 100 and 15'000, most preferably of between 100 and 1'000.

By variation of the concentrations of each component present in the composition, it is possible to alter the resorbtion and mechanical properties of the membrane. For example, the resorption rate may be adapted by the following:

- by choice of an appropriate polyethylene glycol;

- by variation of the ratio of different polyethylene glycols with different average molar masses in a mixture of polyethylene glycols;

- by variation of the amount of polyvinylpyrrolidone;

- by variation of the ratio between the amount of the at least one polyethylene glycol and additive c.

The pressure strength and the swelling characteristics of the membrane may be adapted similarly.

The resorbable membrane is preferably used as cover for burns or wounds. Alternatively, the membrane may also be used to separate organs or tissues during and/or after surgery.

In another alternative embodiment of the present invention a kit comprising a multitude of resorbable membranes according to the present invention with different absorption rates is provided. The absorption rates of each membrane may be adapted by varying the amount of additive c., especially polyvinylpyrrolidone, used in the composition. Preferably the resorption rate may also be varied by the amount of polyole component, especially polycaprolactone used. A kit with a multitude of membranes with different resorption rates allows choosing a membrane with an optimal absorption rate when treating a wound or during surgery. As understood herewith, the term "multitude" means two or more.

Further advantages and characteristics of the present invention are described in the following description of examples and figures.

Fig. 1:    Shows a picture of a resorbable membrane according to the present invention;

Fig. 2:    is a diagram showing the degradation rate of membranes with three different compositions according to the present invention.

Example 1:

Two exemplary formulations of resorbable membranes according to the present invention are given in Table 1. The components are mixed and applied to a silicone film in two different thicknesses (100 and 300 μm) using a wiper. After curing over night at room temperature, the membranes are stored at constant air conditions in a climate room (65% rel. humidity/20°C) at an atmospheric gas composition.

Table 1: Exemplary compositions

| Components | Membrane A [g] | Membrane B [g] | Membrane C [g] |
|---|---|---|---|
| PEG-400 | 8 | 8 | 8 |
| PEG-4000 | 6 | 0 | 12 |
| Sympatens TRH/400 | 0.02 | 0.02 | 0.02 |
| CAPA 4801 | 6 | 12 | 0 |
| Castor Oil | 4 | 4 | 4 |
| Coscat 83 | 0.14 | 0.14 | 0.14 |
| Polyvinylpyrrolidone | 0.84 | 0.84 | 0.84 |
| Ca stearate | 0.5 | 0.5 | 0.5 |
| Sulphonated Castor Oil | 0 | 0 | 0.1 |
| Desmodur E 305 | 16.3 | 16.3 | 16.3 |

First, CAPA 4801 (tetrafunctional polycaprolactone with an average molar mass of 8000, available from Perstorp UK Ltd., Warrington, UK) was melted. In the next step, PEG-400 (Kolb AG, Hedingen, Switzerland) and polyvinylpyrrolidone (Luvitec K30, BASF, Germany) was added and the mixture stirred in a tin cup using the spatula at a temperature of 175°C until a clear solution appeared. Castor oil (Ph. Eur., Alberdingk Boley, Krefeld, Germany) and Sympatens TRH/400 (Kolb AG, Hedingen, Switzerland) as emulsifier were added and the mixture thoroughly stirred using the spatula. Then the mixture was brought to 70°C, transferred into a PP-200 cup (suitable for the SpeedMixer™ system) and Coscat 83 (Vertellus Inc., Indianapolis, USA) and calcium stearate were added. Desmodur E 305 (Bayer Material Science AG, Leverkusen, Germany) pre-heated to 70°C was added to the mixture and stirred using a SpeedMixer™ 400 FVZ (Hauschild & Co KG, Hamm, Germany) for 1 min at 2700 rpm. The resulting composition was spread on siliconized paper with a doctor blade to yield a membrane.

Example 2:

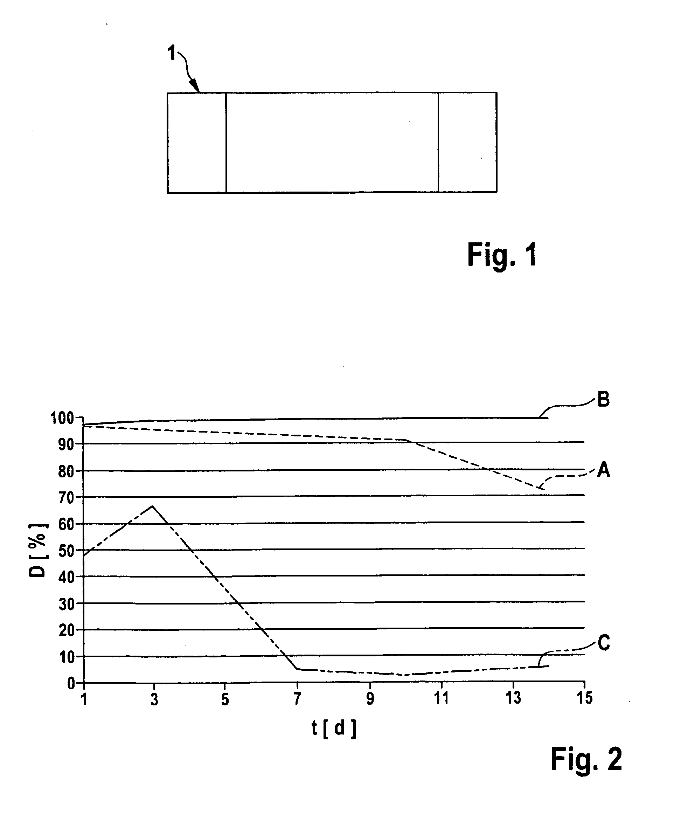

The rate of degradation of the membranes according to example 1 was determined from their time-dependent mass loss. A protocol for the oxidative degradation of polymers in medical products according to ISO 10993-13:1999 was used. A 3% hydrogen peroxide solution served as a medium. The polyurethane membrane samples were dried to constant weight under vacuum. The samples were then placed in a Petri dish containing a 3% hydrogen peroxide solution and incubated at 37°C. During the test, the samples were constantly moved on a laboratory shaker (100 rpm). At various times, the test samples were dried under vacuum to constant weight. The rate of the reaction is correlated to the time dependent weight-loss. The degradation rate of the membranes is adjustable by means of the relative amounts of the components. Figure 2 shows the degradation of membrane A, B and C from example 1 in a time dependent manner.

Example 3:

The moisture vapour transmission rate ("MVTR") of the resorbable polyurethane membranes was measured according to DIN EN 13726-2. Two procedures were used: In procedure 1, a circular test sample of the membrane with a surface of 10cm$^2$ is clamped on a metallic cylinder filled with distilled water up to 5mm below the test sample. The weight of the cylinder assembly is measured. After 18h to 24h of incubation at 37°C the weight of the cylinder is again measured and the MVTR is determined according to the following formula:

$$X = (W_1 - W_2) \times 1000 \times 24 / T$$

where X is the MVTR in g*m$^{-2}$*24h$^{-1}$, $W_1$ and $W_2$ are the determined masses of the cylinder and T is the incubation time in hours.

In the second procedure, the test sample is in contact with the distilled water. This is done by clamping the sample onto the bottom of the closed cylinder.

Membranes A and B were both measured using the thicknesses 100 and 300 micrometers. The average MVTR of the experiments are summarized in table 2.

Table 2: MVTR of resorbable polyurethane membranes

| Experiment | Sample | MVTR in g/m$^2$/24h |
|---|---|---|
| procedure 1 | membrane A (100$\mu$m) | 2064 |
| | membrane A (300$\mu$m) | 1694 |
| | membrane B (100$\mu$m) | 1194 |
| | membrane B (300$\mu$m) | 472 |
| procedure 2 | membrane A (100$\mu$m) | 24708 |
| | membrane A (300$\mu$m) | 19524 |
| | membrane B (100$\mu$m) | 1884 |
| | membrane B (300$\mu$m) | 780 |

As a comparison a commercially available, non-resorbable membrane 'Omiderm™' (available from Taureon, Frankfurt a.M., Germany) with a thickness of 40 $\mu$m, which is mainly used for treatment of burn patients, shows an MVTR of around 5000 g/m$^2$/24h.

Example 4: Surface expansion of polyurethane membranes

The resorbable polyurethane membranes increase in surface after contact with fluids. For documentation purposes polyurethane membrane samples were cut to a surface of 20cm$^2$. The surface expansion was measured after 15 min incubation in distilled water.

Table 3: surface expansion

| Membrane | Dry [cm$^2$] | Wet [cm$^2$] | Relative increase |
|---|---|---|---|
| A (100$\mu$m) | 20.0 | 32.3 | 61.0% |
| A (300$\mu$m) | 20.0 | 33.6 | 68.0% |
| B (100$\mu$m) | 20.0 | 22.1 | 10.5% |
| B (300$\mu$m) | 20.0 | 21.4 | 7.0% |

Example 5: Tensile strength of polyurethane membranes

The tensile strength is an important property for resorbable membranes. The testing on an Instron tensile strength testing equipment involved taking a small sample with a fixed cross-section area (20cm$^2$), and then pulling it with a controlled, gradually increasing force until the sample changes shape or breaks. For 'wet' conditions membrane test samples were incubated for 15 min in distilled water. Table 4 and 5 show the summarized results from analyzed samples under dry and wet conditions.

Table 4: Tensile strength of dry polyurethane membranes

| Sample | Tension at break (MPa) | Elongation at break (%) | Tension at 20% elongation (MPa) | Tension at 40% elongation (MPa) | Tension at 60% elongation (MPa) | Tension at 100% elongation (MPa) |
|---|---|---|---|---|---|---|
| A (100$\mu$m) | 3.298 | 870.4 | 0.858 | 1.050 | 1.135 | 1.235 |
| A (300$\mu$m) | 3.126 | 1074.1 | 0.541 | 0.684 | 0.746 | 0.813 |
| B (100$\mu$m) | 7.714 | 708.8 | 2.185 | 2.518 | 2.683 | 2.897 |
| B (300$\mu$m) | 6.746 | 727.5 | 2.385 | 2.740 | 2.898 | 3.062 |

Table 5: Tensile strength of wet polyurethane membranes

| Sample | Tension at break (MPa) | Elongation at break (%) | Tension at 20% elongation (MPa) | Tension at 40% elongation (MPa) | Tension at 60% elongation (MPa) | Tension at 100% elongation (MPa) |
|---|---|---|---|---|---|---|
| A (100$\mu$m) | 0.038 | 243.1 | 0.085 | 0.111 | 0.129 | 0.148 |
| A (300$\mu$m) | 0.026 | 150.8 | 0.056 | 0.059 | 0.058 | 0.049 |
| B (100$\mu$m) | 2.810 | 312.5 | 1.517 | 1.872 | 2.063 | 2.290 |
| B (300$\mu$m) | 5.722 | 840.9 | 1.826 | 2.250 | 2.445 | 2.660 |

Figure 1 shows a picture of a resorbable membrane 1 according to the present invention. As can be seen, the membrane 1 is transparent, which e.g. allows monitoring the healing process without the need to remove the membrane 1. In this example, the membrane 1 is in the form of a pad. Alternatively, the membrane 1 may be configured in any suitable shape and with any appropriate surface area.

Figure 2 shows a diagram representing the degradation rates of membranes A, B and C from example 1. The horizontal axis represents the loss of mass of the membrane in % of the total mass. As may be seen, the degradation rate may be influenced by varying the amount and the composition of the polyol component. Membrane B has the highest amount of polycaprolactone but no PEG-4000 and exhibits a very low resorbtion rate. On the other hand, Membrane C comprises only PEG-400 and PEG-4000 but no polycaprolactone, which results in a very high resorbtion rate. Membrane A comprises both PEG-4000 and polycaprolactone in a ratio of 1:1, leading to an absorption rate which is higher than that of Membrane B, but still slower than the absorption rate of Membrane C.

## Claims

1.   A resorbable membrane for medical applications, wherein said membrane is permeable to gases but impermeable to other substances, and is produced or producable from a composition comprising:

     a. at least one polyol component comprising at least one compound with at least two, but preferably two, three or four hydroxyl groups;
     b. at least one polyisocyanate component or at least one polyurethane prepolymer comprising at least one compound with at least two, but preferably two, three or four isocyanate groups; and
     c. an additive comprising at least one of the following compounds: polyvinylpyrrolidone, polyvinylpolypyrrolidone, a copolymer of polyvinylpyrrolidone with vinylacetate, vinylimidazole or vinylcaprolactam or mixtures thereof, present in an amount of between 0.001 and 10% by weight, preferably between 1 and 4% by weight.

2.   The resorbable membrane according to claim 1, **characterized in that** the membrane has a thickness of between 10 $\mu$m and 1000 $\mu$m, preferably between 20 $\mu$m and 500 $\mu$m, most preferably between 50 $\mu$m and 300 $\mu$m.

3.   The resorbable membrane according to either of claim 1 or 2, **characterized in that** the at least one polyol component is present in an amount between 1 and 96% by weight, preferably between 30 and 60% by weight, most preferably between 35 and 50% by weight.

4.   The resorbable membrane according to any of claims 1 to 3, **characterized in that** the at least one polyisocyanate component or the at least one polyurethane prepolymer are present in an amount of between 4 and 60% by weight, preferably between 40 and 60% by weight, most preferably between 45 and 55% by weight.

5.   The resorbable membrane according to any of claims 1 to 4, **characterized in that** the polyvinylpyrrolidone has a molecular weight of between 7'000 and 1'000'000 g/mol, preferably between 40'000 and 60'000 g/mol.

6.   The resorbable membrane according to any of claims 1 to 5, **characterized in that** the at least one polyol component comprises at least one polyethyleneglycol.

7.   The resorbable membrane according to any of claims 1 to 6, **characterized in that** the at least one polyol component comprises at least one polycaprolactone, preferably poly-($\varepsilon$-caprolactone).

8.   Kit comprising resorbable membranes according to any of claims 1 to 7 with different resorption rates, **characterized in that** the resorption rates of the membranes are adjusted by the amount of the at least one polyol component a., in particular a polycaprolactone, and/or additive c. used in the composition.

## Patentansprüche

1.   Resorbierbare Membran für medizinische Anwendungen, wobei die Membran für Gase durchlässig, aber für andere Substanzen undurchlässig ist und aus einer Zusammensetzung hergestellt oder herstellbar ist, welche Folgendes umfasst:

     a. mindestens eine Polyolkomponente, umfassend mindestens eine Verbindung mit mindestens zwei, aber vorzugsweise zwei, drei oder vier Hydroxylgruppen;

b. mindestens eine Polyisocyanatkomponente oder mindestens ein Polyurethan-Präpolymer, umfassend mindestens eine Verbindung mit mindestens zwei, aber vorzugsweise zwei, drei oder vier Isocyanatgruppen; und

c. ein Additiv, umfassend mindestens eine der folgenden Verbindungen: Polyvinylpyrrolidon, Polyvinylpolypyrrolidon, ein Copolymer von Polyvinylpyrrolidon mit Vinylacetat, Vinylimidazol oder Vinylcaprolactam oder Mischungen davon, das in einer Menge zwischen 0,001 und 10 Gew.-%, vorzugsweise zwischen 1 und 4 Gew.-% vorliegt.

**2.** Resorbierbare Membran nach Anspruch 1, **dadurch gekennzeichnet, dass** die Membran eine Dicke zwischen 10 $\mu$m 1000 $\mu$m, vorzugsweise zwischen 20 $\mu$m und 500 $\mu$m und ganz besonders bevorzugt zwischen 50 $\mu$m und 300 $\mu$m aufweist.

**3.** Resorbierbare Membran nach Anspruch 1 oder 2, **dadurch gekennzeichnet, dass** die mindestens eine Polyolkomponente in einer Menge zwischen 1 und 96 Gew.-%, vorzugsweise zwischen 30 und 60 Gew.-% und ganz besonders bevorzugt zwischen 35 und 50 Gew.-% vorliegt.

**4.** Resorbierbare Membran nach einem der Ansprüche 1 bis 3, **dadurch gekennzeichnet, dass** die mindestens eine Polyisocyanatkomponente oder das mindestens eine Polyurethan-Präpolymer in einer Menge zwischen 4 und 60 Gew.-%, vorzugsweise zwischen 40 und 60 Gew.-% und ganz besonders bevorzugt zwischen 45 und 55 Gew.-% vorliegt.

**5.** Resorbierbare Membran nach einem der Ansprüche 1 bis 4, **dadurch gekennzeichnet, dass** das Polyvinylpyrrolidon ein Molekulargewicht zwischen 7000 und 1.000.000 g/mol und vorzugsweise zwischen 40.000 und 60.000 g/mol aufweist.

**6.** Resorbierbare Membran nach einem der Ansprüche 1 bis 5, **dadurch gekennzeichnet, dass** die mindestens eine Polyolkomponente mindestens ein Polyethylenglykol umfasst.

**7.** Resorbierbare Membran nach einem der Ansprüche 1 bis 6, **dadurch gekennzeichnet, dass** die mindestens eine Polyolkomponente mindestens ein Polycaprolacton, vorzugsweise Poly($\varepsilon$-caprolacton), umfasst.

**8.** Kit, umfassend resorbierbare Membranen nach einem der Ansprüche 1 bis 7 mit unterschiedlichen Resorptionsraten, **dadurch gekennzeichnet, dass** die Resorptionsraten der Membranen durch die Menge der mindestens einen Polyolkomponente a., insbesondere eines Polycaprolactons, und/oder des Additivs c., die in der Zusammensetzung verwendet werden, eingestellt werden.

## Revendications

**1.** Membrane résorbable destinée à des applications médicales, ladite membrane étant perméable aux gaz mais imperméable à d'autres substances et étant produite ou pouvant être produite à partir d'une composition comprenant:

a. au moins un constituant polyol comprenant au moins un composé contenant au moins deux, mais de préférence deux, trois ou quatre groupes hydroxyle;

b. au moins un constituant polyisocyanate ou au moins un prépolymère de polyuréthane comprenant au moins un composé contenant au moins deux, mais de préférence deux, trois ou quatre groupes isocyanate; et

c. un additif comprenant au moins un des composés suivants: une polyvinylpyrrolidone, une polyvinylpolypyrrolidone, un copolymère de polyvinylpyrrolidone avec de l'acétate de vinyle, du vinylimidazole ou du vinylcaprolactame ou des mélanges correspondants, présent en une quantité entre 0,001 et 10% en poids, de préférence entre 1 et 4% en poids.

**2.** Membrane résorbable selon la revendication 1, **caractérisée en ce que** la membrane présente une épaisseur entre 10 $\mu$m et 1000 $\mu$m, de préférence entre 20 $\mu$m et 500 $\mu$m, le plus préférablement entre 50 $\mu$m et 300 $\mu$m.

**3.** Membrane résorbable selon l'une ou l'autre de la revendication 1 ou 2, **caractérisée en ce que** ledit au moins un constituant polyol est présent en une quantité entre 1 et 96% en poids, de préférence entre 30 et 60% en poids, le plus préférablement entre 35 et 50% en poids.

**4.** Membrane résorbable selon l'une quelconque des revendications 1 à 3, **caractérisée en ce que** ledit au moins un constituant polyisocyanate ou ledit au moins un prépolymère de polyuréthane sont présents en une quantité entre 4 et 60% en poids, de préférence entre 40 et 60% en poids, le plus préférablement entre 45 et 55% en poids.

**5.** Membrane résorbable selon l'une quelconque des revendications 1 à 4, **caractérisée en ce que** la polyvinylpyrrolidone présente un poids moléculaire entre 7000 et 1 000 000 g/mole, de préférence entre 40 000 et 60 000 g/mole.

**6.** Membrane résorbable selon l'une quelconque des revendications 1 à 5, **caractérisée en ce que** ledit au moins un constituant polyol comprend au moins un polyéthylèneglycol.

**7.** Membrane résorbable selon l'une quelconque des revendications 1 à 6, **caractérisée en ce que** ledit au moins un constituant polyol comprend au moins une polycaprolactone, de préférence la poly-($\varepsilon$-caprolactone).

**8.** Kit comprenant des membranes résorbables selon l'une quelconque des revendications 1 à 7 dotées de vitesses de résorption différentes, **caractérisé en ce que** les vitesses de résorption des membranes sont ajustées par la quantité dudit au moins un constituant polyol a., en particulier une polycaprolactone, et/ou de l'additif c., utilisés dans la composition.

**Fig. 1**

**Fig. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 19940977 A1
- US 6706058 B
- WO 0167987 A
- WO 9947072 A1

### Non-patent literature cited in the description

- Topical Treatment of Toxic Epidermal Necrolysis using Omiderm® and Glycerol-Preserved Human Cadaver Skin. **ACIKEL C. ; EREN F. ; ERGUN O. ; CELIKOZ B.** Annals of Burns and Fire Disasters. June 2002, vol. XV
- **UHLIG C. ; RAPP M. ; HARTMANN B. ; HIERLEMANN H. ; PLANCK H. ; DITTEL K.-K.** Suprathel® - An innovative, resorbable skin substitue for treatment of burn victims. *Burns,* 2007, vol. 33, 221-229
- **KROEZE R.J. ; HELDER M.N. ; GOVAERT L.E. ; SMIT T.H.** Biodegradable Polymers in Bone Tissue Engineering. *Materials,* 2009, vol. 2, 833-856
- **S.A. GUELCHER.** Biodegradable Polyurethanes: Synthesis and Applications in Regenerative Medicine. *Tissue engineering: Part B,* 2008, vol. 14 (1
- **E.M. CHRISTENSON ; S. PATEL ; J.M. ANDERSON ; A. HILTNER.** Enzymatic Degradation of Poly(ether urethane) and poly (carbonate urethane) by cholesterol esterase. *Biomaterials,* 2006, vol. 23, 3920-3926